Europäisches Patentamt

European Patent Office

Office européen des brevets

(11) Numéro de publication: **0 513 442 A1**

## DEMANDE DE BREVET EUROPEEN

(21) Numéro de dépôt: 91201049.3

(22) Date de dépôt: 16.05.91

(51) Int. Cl.⁵: **A61K 31/04**

(43) Date de publication de la demande:
**19.11.92 Bulletin  92/47**

(84) Etats contractants désignés:
**NL**

(71) Demandeur: **Kowalski, Romuald, Dr.**
**UPT Wroclaw 2 Poste restante ul.**
**Malachowskiego 1**
**Wroclaw(PL)**

(72) Inventeur: **Kowalski, Romuald, Dr.**
**UPT Wroclaw 2 Poste restante ul.**
**Malachowskiego 1**
**Wroclaw(PL)**

(74) Mandataire: **Roman, Michel**
**CABINET ROMAN 35, rue Paradis**
**F-13001 Marseille(FR)**

(54) Composition contenant du sulfate de barium pour le traitement des hémorroides.

(57) Le médicament Laitvit - Hémorroïdes - R.K., grâce à la teneur en substance active - sulfate de baryum, est très bon pour le traitement des états inflammatoires du rectum ainsi que des hémorroïdes de cette région compliquées ou non par les états inflammatoires.

Ce médicament rend des services inappréciables dans le traitement des prurits anaux de toute sorte ainsi que des fistules rectales surtout en ce qui concerne celles d'origine mécanique.

En résultat de ce traitement plusieurs hémorroïdes et fistules rectales disparaissent ou subissent une sensible diminution et les interventions chirurgicales s'avèrent inutiles.

D'autres propriétés antitumorigènes sont utilisées pour détruire les excroissances carcinomateuses dans les cas des recto-carcinomes inopérables.

EP 0 513 442 A1

1 - ère partie

Le médicament Laitvit - hémorroïdes - R.K. vu ses propriétés particulières à savoir :

1. - anti inflammatoires,
2. - analgétiques et
3. - antipruritiques

est très bon pour soigner hémorroïdes et les états inflammatoires du rectum/proctites.

Ce médicament peut être appliqué sous la forme de :

1. - suppositoires intra-rectaux,
2. - pommade intra-rectable,
3. - traitement combiné - péroral et rectable

Sous l'effet d'un des modes de traitement indiqués ci-dessus, les nodules hémorroïdes intérieurs et extérieurs disparaissent ou diminueront sensiblement.

Le prurit et la proctite disparaissent eux aussi.

La défectation indolore est d'habitude régulière.

Si ce sont les proctites ou les nodules hémorroïdes changés sous l'effet de l'inflammation qui étaient la cause de la constipation celle-ci disparait Le médicament Laitvit - Hémorroïdes - R.K. agit comme un baume calmant sur le segment inférieur du boyau fécal.

D'habitude de petits nodules hémorroïdes disparaissent déjà au bout de 3 mois de traitement, de grands nodules négligés demandent un traitement d'un an ou moins et l'application d'un traitement préventif.

Aucun âge ne constitue une contre indication au traitement des hémorroïdes du rectum.

D'habitude il n'y a pas non plus de contre indication à l'application du Laitvit - Hémorroïdes - R.K. en égard à d'autres affections accompagnant les nodules hémorroïdes.

Le traitement des hémorroïdes avec le médicament Laitvit - Hémorroïdes - R. K. a sauvé beaucoup de patients d'une intervention chirurgicale mutilante.

Le médicament Laitvit - Hémorroïdes - R.K.

2 - ème partie

Description des cas particuliers /a/

1. - Cas N° 1/G.A./ - une femme de 54 ans s'était présentée en octobre 1990 afin de subir un traitement avec le médicament Laitvit - Hémorroïdes - R.K. à cause des hémorroïdes.

C'est depuis 15 ans qu'elle souffre à cause d'hémorroïdes. Le médecin qui l'avait examinée autrefois avait décelé les hémorroïdes sortant à l'extérieur douloureuses à toucher.

Les selles étaient constipées et très douloureuses.

Leur endolorissement augmentait quand il faisait froid, quand elles prenaient froid.

Au bout d'un mois de traitement déjà la patiente a éprouvé un sensible soulagement :

- les selles sont devenues moins douloureuses et plus fréquentes.

Après trois mois de traitement avec le médicament Laitvit - Hémorroïdes - R.K. - l'amélioration ultérieure s'est produite :

- les selles sont devenues régulières et quotidiennes,
- les hémorroïdes ont sensiblement diminué, elles ne saignaient plus et étaient indolores.

Au fur et à mesure du traitement ultérieur l'état du segment inférieur de l'intestin gros améliorait de plus en plus, la patiente ne se plaignait plus de l'affection du côté des hémorroïdes qui ont disparu et ne faisaient plus mal. Depuis ce moment là, la malade prenait ce médicament en dose préventive.

L'examen de contrôle fait le 09.05.86 c'est-à-dire au bout de 2 à 6 mois de traitement a permis de déceler la disparition des hémorroïdes.

Qaut aux selles, elles sont devenues régulières et quotidiennes.

L'état général de santé de la patiente est bon.

2. Cas N° 2/S.G./ - Une femme de 54 ans s'était présentée au mois de février 1984 afin de subir un traitement avec le médicament Laitvit - Hémorroïdes - R.K. à cause de :

- hémorroïdes,
- constipation,
- état post-cholecytectomie,
- névrose généralisée et épuisement sensible

C'est depuis six ans qu'elle souffre à cause d'hémorroïdes. L'examen du colon fait au Service Endoscopique de l'Académie de Médecine de WROCLAW, le 30.03.84 a permis de déceler :

- hémorroïdes dans la région extérieure de l'anus
- tonus/tension/du sphincter bonne.

L'endoscope a été introduit à la profondeur de 15 cm.

La muqueuse de ce segment était injectée, lisse et luisante.

Le sphincter interne injecté avec les hémorroïdes à l'intérieur.

On avait recommandé une intervention chirurgicale que la patiente avait refusée.

La patiente se plaignait de selles douloureuses et constipées tous les 3 à 4 jours mélangées souvant avec du sang.

L'une des hémorroïdes était grosse comme une prune et deux autres un peu plus petites.

Au bout d'un mois de traitement avec le médicament Laitvit - Hémorroïdes - R.K. - La patiente avait éprouvé un grand soulagement.

Les selles sont devenues moins douloureuses et le saignement s'est presque arrêté.

Après 3 mois de traitement avec le médicament Laitvit - Hémorroïdes - R.K. - l'état de santé de la patiente s'est encore amélioré davantage :
- les selles sont devenues indolores et
- le saignement s'est tout à fait arrêté,
- les hémorroïdes se sont sensiblement diminuées
- elles ne salissaient plus au dehors

L'examen de contrôle fait le 9.05.85 c'est-à-dire au bout d'un et deux mois de traitement a permis de déceler une diminution des hémorroïdes jusqu'à 2/3 de leur volume précédant.

Elles sont devenues indolores, molles et ne saignaient plus, les selles sont devenues régulières et indolores.

L'état général de santé de la patiente s'est sensiblement amélioré.

Ainsi la patiente a évité une intervention chirurgicale.

3. - Cas N° 3 - une femme de 55 ans s'était présentée en mai 1983 pour se faire soigner avec le médicament Laitvit - Hémorroïdes - R.K. à cause de :
- hémorroïdes et
- état anxieux et maux de tête

Les hémorroïdes, elle en souffre depuis 30 ans, et elles sont accompagnées des constipations.

La patiente ne pouvait pas évacuer les selles sans remèdes auxiliaires.

Les hémorroïdes : l'une d'elles était grosse comme une prune, les autres étaient plus petites. Elles étaient douloureuses, salissaient à l'extérieur et saignaient.

Après un mois de traitement avec Laitvit - Hémorroïdes - R.K. - une amélioration très sensible s'est produite :
- les selles sont devenues indolores et
- moins constipées.

Au bout d'un an de traitement on a constaté une sensible amélioration :
- l'hémorroïde grosse comme une prune a diminué jusqu'au volume d'une cerise,
- les autres ont disparu,
- les selles sont devenues régulières et indolores.

L'examen de contrôle fait le 06.05.1986 a permis de constater une sensible amélioration :
- les selles sont devenues régulières, indolores , et
- elles ne saignaient plus,
- seule l'hémorroïde grosse comme une cerise persiste encore mais elle est indolore et ne saigne plus.

L'état de santé de la patiente est satisfaisant.

Ainsi la patiente a évité une intervention chirurgicale.

Le médicament Laitvit - Hémorroïdes - R.K.

2 ème partie

Description des cas particuliers:b/

4. - Cas N° 4/S.K./ - une femme de 63 ans s'était présentée en octobre 1983 pour se faire soigner avec le médicament Laitvit - Hémorroïdes - R.K. à cause des hémorroïdes.

Les hémorroïdes, elle en souffre depuis 5 ans.

Dans le rectum on a décelé un tubercule gros comme le grain de haricot, douloureux et pruriteux.

Les selles étaient constipées tous les 3 à 4 jours, souvent teintées du sang frais.

On lui recommandait une intervention chirurgicale mais elle ne l'a pas acceptée.

Au bout de deux semaines déjà de traitement avec du Laitvit - Hémorroïdes - R.K. la patiente a éprouvé un sensible soulagement.

Les douleurs accompagnant la défécation se sont sensiblement adoucies, les défécations sont devenues plus régulières.

Après un mois de traitement combiné le tubercule hémorroïdal a disparu, les selles sont devenues régulières et indolores.

La patiente continuait encore toujours le taitement avec le médicament Laitvit - Hémorroïdes - R.K.

L'examen de contrôle fait le 06.05.85 c'est-à-dire après 2 et 3 mois de traitement préventif avec le médicament Laitvit - Hémorroïdes - R. K., a permis de déceler une image régulière du rectum.

Les hémorroïdes ont disparu, les selles sont devenues régulières et quotidiennes.

L'état général de santé de la patiente s'est aussi sensiblement améliore.

5.- Cas N° 5 - P.W./ - une femme de 60 ans s'était présentée le 25.01.84 pour se faire soigner avec le médicament Laitvit - Hémorroïdes - R.K., à cause des hémorroïdes.

Les hémorroïdes saignantes grosses comme une prune, la patiente en souffre depuis trois ans. De plus, le chirurgien présumait l'existance d'une fistule périrectale.

Comme le traitement poursuivi jusqu'à présent, ce temps là, ne donnait aucun résultat positif, on lui a recommandé une intervention chirurgicale que la patiente a refusée.

Les hémorroïdes étaient accompagnées , les constipations, défécations tous les 3 à 4 jours et encore très douloureuses.

Déjà au bout d'un mois du traitement une sensible amélioration s'est fait remarquer:
- les selles sont devenues indolores,
- moins constipées et plus régulières.

Le bien être général de la patiente; lui aussi s'est sensiblement améliore.

Au bout de 3 mois de traitement l'amélioration ultrieure de santé de la patiente s'est produite :

- les selles sont devenues régulières et
- indolores,
- les saignements des hémorroïdes se sont arrêtés,
- les hémorroïdes sont devenues plus molles, plus petites et indolores.

au bout de six mois de traitement l'hémorroïde grosse comme une prune s'est diminuée jusqu'au volume d'une cerise et elle est devenue indolore.

Les selles étaient régulières et indolores.

L'état général de santé de la patiente s'est aussi sensiblement amélioré.

L'examen de contrôle fait le 4.05.1986 c'est-àdire au bout de 2 ans et 3 mois de traitement a permis de déceler la normalisation ultérieure de l'image du rectum.

L'hémorroïde s'est davantage diminuée, elle est indolore ne saigne plus et les selles sont devenues régulières.C'est seulement quand la patiente prend froid, elle éprouve de petites affections qui pourtant disparaissent dès qu'elle se réchauffe.

L'état général de santé de la patiente est satisfaisant.

6. - Cas N° 6 - une femme de 61 ans s'est présentée pour se faire soigner avec le médicament Laitvit - Hémorroïdes - R.K. à cause de : hémorroïdes et ulcères variqueux sur deux cuisses.

Quant aux hémorrodïdes elle dit qu'elle en souffre depuis quelques années que les selles sont constipées tous les 2 à 3 jours, douloureuses.

Au bout d'un mois de traitement avec Laitvit - Hémorroïdes - R. K. - la patiente a éprouvé un grand soulagement :
- les selles sont devenues indolores, et régulières.

L'examen de contrôle fait le 08.0507986 c'est-à-dire après 6 mois de traitement a permis de déceler la disparition des tubercules hémorroïdaux et les selles sont devenues indolores et régulières.

L'état général de santé lui aussi s'est sensiblement amélioré.

7. - Cas N° 7/J.T./ - une femme de 54 ans s'était présentée en janvier 1986 pour se faire soigner avec du Lairvit - Hémorroïdes - R. K. à cause de :

1. - hémorroïdes et
2. - oedème de la jambe droite.

Les hémorroïdes, la patiente en souffre depuis quelques années.

C'étaient les hémorroïdes en chou-fleur, salissent en dehors du sphincter externe de l'anus, très douloureuses et saignantes.

Les selles étaient très douloureuses tous les 3 jours.

En résultat du traitement avec du Laitvit - Hémorroïdes - R. K.:
- les hémorroïdes ont sensiblement diminué
- elles sont devenues indolores et

- les selles indolores et assez régulières

L'examen de contrôle fait le 06.05.1986 c'est-à-dire au bout de quatre mois de traitement a décelé une amélioration très importante :
- les hémorroïdes ont sensiblement diminué
- elles ne saignent plus et sont indolores,
- les selles sont devenues régulières et indolores

L'état général de santé de la patiente est très bon

8. - Cas N° 8/R.A./ - une femme de 63 ans s'était présenté au traitement en octobre 1983 et c'est à cause des hémorroïdes et d'affection hépato - vésiculaires.

C'est depuis plusieurs années qu'elle souffre à cause d'hémorroïdes internes, qui ont apparu par la suite des grossesses et qui étaient très douloureuses.

Les selles étaient constipées et douloureuses.

Déjà au bout d'un mois de traitement avec du Laitvit - Hémorroïdes - R.K. la patiente a éprouvé un sensible soulagement.
- les selles sont devenues indolores
- et régulières.

Après trois mois de traitement une amélioration ultérieure s'est prouduite.

Au bout d'un an de traitement la patiente ne se plaint d'affection du côté des hémorroïdes :
- les selles sont devenues régulières et indolores
- les hémorroïdes sont devenues impalpables.

L'examen de contrôle fait en mai 1986 c'est-à-dire après 2 ans et 7 mois, de traitement préventif n'a plus décelé d'hémorroïdes,
- les selles sont régulières et indolores.

L'état général de santé de la patiente s'est considérablement amélioré : elle se sent en bonne santé et fort.

Elle se plaint d'aucune affection.

9. - N° 9 /O.L./ - une femme de 70 ans s'était présentée en octobre 1983 pour se faire soigner avec le médicament Laitvit - Hmérroïdes - R. L. à cause de : hémérroïdes. C'est depuis 5 ans qu'elle souffre d'hémorroïdes.
- les selles étaient constipées et
- douloureuses,
- parfois mélangées du sang

Déjà au bout d'un mois de traitement une sensible amélioration s'est produite:
- les selles sont devenues indolores et
- régulières,

Au bout de 3 mois de traitement on pouvait remarquer l'amélioration ultérieure :
- la patiente ne se plaignait plus de douleurs accompagnant la défecation et
- les selles sont devenues régulières et quotidiennes.

Au bout d'un an et six mois de traitement :

- les hémorroïdes ont disparu
- les selles étaient régulières quotiennes et indolores

L'état général de santé de la patiente était très bon.

Le contrôle fait le 21.05.1986 c'est-à-dire après presque 3 ans de traitement a décelé une image régulière du rectum :

- les hémorrodïdes Ont disparu
- les selles sont régulières, indolores et quotïdiennes.

L'état général de santé de la patiente est parfait.

**Revendications**

1. Le médicament Laitvit - Hémorroïdes - R.K. contient la substance active - sulfate de baryum

2. Cette substance possède les propriétés :
   a/- anti-inflammatoires,
   b/- analgétiques,
   c/- antipruritiques et
   d/- antitumorigènes.

3. Les propriétés thérapeutiques de cette substance mentionnées dans les points 1 et 2 sont parfaites pour le traitement de :
   1.- hémorroïdes,
   2.- proctitis,
   3.- fistules rectales compliquées ou non par les états inflammatoires surtour de ceux d'origine mécanique
   4.- pour mitiger le prurit anal de toute sorte.
   5.- pour détruire les excroissances carcinomateuses dans les cas inopérables de ce segment du rectum.

Office européen
des brevets

**RAPPORT DE RECHERCHE EUROPEENNE**

Numero de la demande

EP    91 20 1049

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| E | EP-A-0 451 300 (KOWALSKI, ROMUALD) 16 Octobre 1991 <br> * abrégé * <br><br> --- | 1-5 | A61K31/04 |
| X | FR-A-2 639 829 (KOWALSKI, ROMUALD) 8 Juin 1990 <br> * abrégé * <br><br> --- | 1-5 | |
| X | US-A-4 477 439 (GAETANO F. D'ALELIO) 16 Octobre 1984 <br> * revendications * <br><br> ----- | 1-5 | |

|  |  |
|---|---|
| | **DOMAINES TECHNIQUES RECHERCHES (Int. Cl.5 )** |
| | A61K |

Le présent rapport a été établi pour toutes les revendications

| Lieu de la recherche | Date d'achèvement de la recherche | Examinateur |
|---|---|---|
| LA HAYE | 11 SEPTEMBRE 1992 | LEHERTE C. F.M. |